# EUROPÄISCHE PATENTSCHRIFT

(11) **EP 2 987 781 B1**
(45) Veröffentlichungstag und Bekanntmachung des Hinweises auf die Patenterteilung: **09.10.2019**
(21) Anmeldenummer: 15002061.8
(22) Anmeldetag: 09.07.2015
(51) Int. Cl.: C07C 41/56, C10L 1/185

(54) **VERFAHREN ZUR HERSTELLUNG VON OXYMETHYLENDIALKYLETHERN UND DEREN VERWENDUNG**
METHOD FOR THE PREPARATION OF OXYMETHYLENE DIALKYL ETHERS AND THEIR USE
PROCEDE DE FABRICATION D'ETHERS D'OXYMETHYLENDIALYLE LEUR UTILISATION

(30) Priorität: 22.08.2014 DE 102014112021
(43) Veröffentlichungstag der Anmeldung: 24.02.2016
(73) Patentinhaber: Karlsruher Institut für Technologie, 76131 Karlsruhe (DE)
(72) Erfinder: Arnold, Ulrich, 76646 Bruchsal (DE); Lautenschütz, Ludger, 76344 Eggenstein-Leopoldshafen (DE); Oestreich, Dorian, 76227 Karlsruhe (DE); Sauer, Jörg, 76135 Karlsruhe (DE)

(56) Entgegenhaltungen:
- EP-A2- 0 150 280
- WO-A1-2006/045506

## Beschreibung

Die vorliegende Erfindung betrifft ein Verfahren zur Herstellung von Oxymethylendialkylethern.

Oxymethylendialkylether (OMDAE), insbesondere Oxymethylendimethylether (OMDME, CH₃O-(CH₂O)ₙ-CH₃, n ≈ 1 - 6) sind vielversprechende Kraftstoffadditive zur Rußminderung in Dieselmotoren. Aufgrund ihres Sauerstoffgehalts unterbinden sie bereits im Verbrennungsstadium die Bildung von Schadstoffen. Damit können die immer rigoroser werdenden Abgasnormen erfüllt werden, bei gleichzeitiger Vereinfachung der Abgasnachbehandlung. Weiterhin kann der Luftbedarf des Motors und damit die Ladungswechselarbeit verringert werden, was zu einer Verbesserung des Wirkungsgrades des Motors führen kann. OMDME weisen günstige physikalisch-chemische und verbrennungstechnische Eigenschaften, wie z.B. hohe Cetanzahlen, auf. Sie sind ungiftig, nicht korrosiv und mit gängigen Kraftstoffen gut mischbar. Ein weiterer Vorteil ist, dass sie aus Methanol gewonnen werden können. Da Methanol seinerseits aus nachwachsenden Rohstoffen und Reststoffen gewonnen werden kann, wird damit nicht nur eine Senkung von CO₂-Emissionen, sondern auch eine NOₓ- und Partikelemissionsminderung im Sinne einer ganzheitlichen Lösung erreicht. Darüber hinaus ist die Gewinnung von OMDME aus sauerstoffhaltigen Ausgangsstoffen besonders effizient, da der Sauerstoff weitgehend im Produkt verbleibt und nicht, wie dies bei der Erzeugung einiger Biokraftstoffe der Fall ist, mit erheblichem Aufwand entzogen werden muss. Dies führt zu einer hohen Atom- und Energieeffizienz im Herstellungsprozess.

Trotz dieser Vorzüge stellt die wirtschaftliche Gewinnung von OMDME immer noch eine Herausforderung dar. Das in WO 2006045506 A1 beschriebene Verfahren, die wasserfreie Umsetzung von Dimethoxymethan (DMM) mit Trioxan, ist mit erheblichem Aufwand verbunden. Entscheidende Nachteile des Verfahrens sind die vergleichsweise teuren Ausgangsstoffe und die umfangreiche Aufarbeitung des Produktgemischs in einem mehrstufigen Destillationsverfahren.

In DE 10 2005 027 702 A1 ist ein Verfahren zur Herstellung von OMDME aus Methanol und wässriger Formaldehydlösung beschrieben. Dabei entstehen unerwünschte Nebenprodukte wie z.B. Halbacetale und Glykole, die aufgrund ähnlicher Siedepunkte nur schwer von OMDME destillativ abtrennbar sind. Das Produktgemisch wird deshalb aufwändig mit Hilfe von zahl-reichen in Serie geschalteten Rektifikationskolonnen und einem Wasserabscheider aufgetrennt, und es werden auf diese Weise OMDME des Typs CH₃O-(CH₂O)ₙ-CH₃ mit n = 3 und 4 gewonnen.

DE 2 163 907 A beschreibt die Herstellung von OMDME aus Methanol und unterschiedlichen Formaldehydquellen. Das im Reaktionsgemisch vorhandene Wasser, das über Formalin eingebracht wird bzw. in der Reaktion entsteht, wird durch azeotrope Destillation mit Hilfe von Cyclohexan entfernt. Cyclohexan dient zusätzlich als Extraktionsmittel, das in einem weiteren Verfahrensschritt vom gewünschten Produkt destillativ abgetrennt werden muss. Nachteilig ist die, im Vergleich zum hier vorgeschlagenen Prozess, komplexe Verfahrensweise. Ein weiterer Nachteil ist, dass sich Formalin verhältnismäßig gut in Cyclohexan löst (siehe Tabelle 1), was die anschließende Trennung erschwert.

EP 0 150 280 A2 offenbart die Herstellung von Acetalen durch Umsetzung von Aldehyden mit Alkoholen in flüssiger Phase und deren Extraktion mit Wasser und mit Wasser nicht mischbarer Lösungsmitteln, wobei die Acetale und nicht umgesetzte Anteile der Aldehyde und Alkohole aus den Extrakten z.B. per Destillation gewonnen werden.

Die bekannten Prozesse auf Basis von Methanol und Formaldehydproduzierenden Verbindungen vereint, dass sie wenig selektiv sind, d.h. sie führen zu komplexen Produktgemischen, die auch zahlreiche unerwünschte Komponenten enthalten. Aufgrund der geringen Selektivität sind hochaufwändige Destillationsschritte zur Produktaufarbeitung notwendig.

Aufgabe der vorliegenden Erfindung ist es, ein Verfahren bereitzustellen, das die Nachteile des Standes der Technik überwindet. Insbesondere liegt der vorliegenden Erfindung die Aufgabe zugrunde, ein Verfahren zur Herstellung von Oxymethylendialkylethern (OMDAE) vorzuschlagen, das sich im Unterschied zum Stand der Technik einfach, technisch unaufwändig und reproduzierbar anwenden lässt. Insbesondere soll ein Verfahren gezeigt werden, welches es ermöglicht Dieseladditive direkt in dem Kraftstoff, indem Sie angereichert werden sollen, zu synthetisieren.

Diese Aufgabe wird im Hinblick auf das Verfahren durch die Merkmale des Anspruchs 1 gelöst. Die Unteransprüche beschreiben jeweils vorteilhafte Ausgestaltungen der Erfindung.

Diese Aufgabe wird durch ein Verfahren zur Herstellung oligomerer OMDAE mit den folgenden Verfahrensschritten gelöst:
a) Bereitstellen mindestens einer Komponente A und mindestens einer Komponente B, wobei A ein Alkohol und/oder eine Carbonsäure und B ein Aldehyd und/oder ein Keton ist,
b) Umsetzung der mindestens einen Komponenten A und B mit einem sauren Katalysator,
c) Erzeugung einer wässrigen und einer organischen Phase,
d) Entnahme der organischen Phase,
wobei in Verfahrensschritt c) als Extraktionsmittel mindestens Diesel hinzugegeben wird.

Der Begriff Oxymethylendialkylether (OMDAE) beschreibt im Folgenden Verbindungen mit der allgemeine Formel R¹O-(CR³R⁴-O)ₙ-R² (I) mit R¹, R² = Alkyl, Cycloalkyl, Acyl und R³, R⁴ = Alkyl, Cycloalkyl, Wasserstoff, wobei die Reste R¹, R², R³ und/oder R⁴ in einer Ausgestaltung Heteroatome aufweisen, und R¹ =/≠ R² =/≠ R³ =/≠ R⁴ und mit n = ganze Zahl ≥ 1.

Im Rahmen der vorliegenden Erfindung bedeutet der Begriff "Raumtemperatur" eine Temperatur von 16 bis 25 °C. Sofern nichts anderes angegeben wird, werden die angeführten Reaktionen bzw. Verfahrensschritte bei Normaldruck/ Atmosphärendruck, d.h. bei 1013 mbar, durchgeführt.

Die Abkürzung "v/v" bedeutet im Rahmen der vorliegenden Erfindung "Volumen der betreffenden Komponente je Gesamtvolumen des Gemischs".

Im Rahmen der vorliegenden Erfindung umfasst der Begriff "Diesel" ein Gemisch aus organischen Verbindungen gemäß DIN EN 590.

Im Rahmen der vorliegenden Erfindung schließt die Formulierung "und/oder" sowohl jede beliebige als auch alle Kombinationen der in der jeweiligen Auflistung genannten Elemente ein.

Ausgestaltungen können grundsätzlich frei miteinander kombiniert werden.

Die Erfindung beinhaltet sowohl verfahrenstechnische als auch chemische Aspekte zur verbesserten Gewinnung von oligomeren Oxymethylendialkylethern (OMDAE) unter der Verwendung einfacher Ausgangsstoffe, wie Alkohole und/oder Carbonsäuren und Aldehyde und/oder Ketone, und die Produktion in einem wässrigen Reaktionsmedium. Herkömmliche Verfahren führen dabei zur Bildung unerwünschter Nebenprodukte wie z.B. Halbacetale oder Glykole, die, wie auch die im Reaktionsgemisch verbleibenden Ausgangsstoffe, wie z.B. bei der Herstellung von OMDME Methanol, Formaldehyd und Wasser, mit einigem Aufwand abgetrennt werden müssen. Besonders die entstehenden Halbacetale sind aufgrund vergleichbarer Siedepunkte nur sehr schwer von OMDME destillativ abtrennbar. Des Weiteren ist die Reaktion zwar vergleichsweise schnell aber gleichgewichtslimitiert. Dadurch kann nur begrenzt Einfluss auf die Zusammensetzung des Produktgemischs ausgeübt werden. Ein weiteres Hindernis stellen Polymerisationsreaktionen des Formaldehyds dar. Diese können unter bestimmten Reaktionsbedingungen zu Polyoxymethylenen (POM) führen. POM stellen bei der Synthese oligomerer OMDME lediglich unerwünschte Nebenprodukte dar, die zur Verstopfung, von Anlagenteilen führen können.

Das erfindungsgemäße Verfahren eignet sich zur Herstellung oligomerer Oxymethylendialkylether (OMDAE) mit der Formel

R¹O-(CR³R⁴-O)ₙ-R² (I)

wobei R¹, R² = Alkyl, Cycloalkyl, Acyl und R³, R⁴ = Alkyl, Cycloalkyl, Wasserstoff sind, wobei die Reste R¹, R², R³ und/oder R⁴ Heteroatome aufweisen können, und R¹, R², R³ und R⁴ unabhängig voneinander gleich oder verschieden sind und n eine ganze Zahle ≥ 1. In einer bevorzugten Ausgestaltung sind R¹ und R² = Methyl, Ethyl. In einer bevorzugten Ausgestaltung ist R³ und R⁴ Wasserstoff. In einer weiteren Ausgestaltung entsprechen R³ und/oder R⁴ eines Moleküls verschiedenen Resten. In einer weiteren Ausgestaltung entspricht n den Zahlen 1, 2, 3, 4, 5 und/oder 6.

Mindestens eine Komponente A, ein Alkohol und/oder eine Carbonsäure, und mindestens eine Komponente B, ein Aldehyd und/oder ein Keton, werden in einem ersten Verfahrensschritt a) bereitgestellt und anschließend zusammengeführt und in Anwesenheit mindestens eines sauren Katalysators umgesetzt (Verfahrensschritt b).

Die Umsetzung erfolgt nach dem folgenden allgemeinen Reaktionsschema:

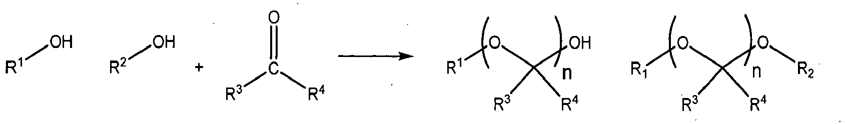

Im Speziellen ist im folgenden Reaktionsschema die Herstellung der Oxymethylendimethylether (OMDME) gezeigt:

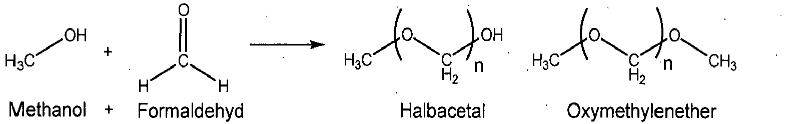

Als Katalysatoren werden homogene oder heterogene saure Katalysatoren eingesetzt. Besonders Mineralsäuren, Heteropolysäuren, Zeolithe, saure Ionenaustauscherharze, Aluminosilikate, Aluminiumoxid, Titandioxid, Zirkondioxid sowie Schwefelsäure und Sulfonsäuren sind geeignete Katalysatoren. Die Katalysatormenge liegt im Bereich von mindestens 0,05 Gew.-%, bevorzugt von 0,5 Gew.-% und maximal 10 Gew.-%, bevorzugt maximal 1 Gew.-%, bezogen auf die Gesamtmasse der Edukte im Reaktionsgemisch.

Die Reaktionstemperatur liegt zwischen 30 °C und 150 °C, bevorzugt zwischen 50 °C und 80 °C. Der optimale Prozessdruck liegt zwischen 1 und 10 bar bevorzugt bei 1 bis 3 bar. Das molare Verhältnis von Komponente B zu Komponente A beträgt 0,5 zu 1 bis 10 zu 1, bevorzugt 1,5 zu 1 bis 3 zu 1. Sollten mehr als eine Komponente A und/oder B eingesetzt werden, bezieht sich das molare Verhältnis auf die Gesamtmenge der Komponenten A zu der Gesamtmenge der Komponenten B. Die Reaktionszeit beträgt 0,1 bis 30 h, bevorzugt 0,5 bis 3 h.

Die Herstellung wird in einer Ausgestaltung in einem Batch-Verfahren durchgeführt, in einer weiteren Ausgestaltung als kontinuierliches Verfahren. Die Umsetzung findet bevorzugt in einem kontinuierlichen Rührkessel oder einem Rohrreaktor statt. Im kontinuierlichen Verfahren werden bevorzugt saure Feststoffsäuren, wie Ionenaustauscherharze und Zeolithe, als Katalysatoren in einem Festbettreaktor eingesetzt.

Das resultierende Reaktionsgemisch wird im Verfahrensschritt c) mit mindestens Diesel als Extraktionsmittel versetzt, wodurch sich eine organische und eine wässrige Phase ausbilden. Die organische Phase wird dann dem Reaktionsgemisch entnommen. Das erfolgt in einer Ausgestaltung in einem Phasenseparator oder einem Mischabsetzer. Die organische Phase setzt sich aus dem mindestens einen während der Synthese entstandenen OMDAE und dem Extraktionsmittel zusammen.

Dabei kommt es zur Ausbildung zweier klar unterscheidbarer Phasen und die erwünschten OMDAE gehen selektiv in die organische Phase über. Nebenprodukte, wie Halbacetale und Glykole und nicht umgesetzte Einsatzstoffe, die Komponenten A und B und Wasser, verbleiben in der wässrigen Phase und können auf diese Weise sehr einfach und nahezu vollständig abgetrennt werden. Zur Ermittlung geeigneter Extraktionsmittel wurde ein breites Lösungsmittelscreening durchgeführt (Tabelle 1). Dabei haben sich solche Lösungsmittel besonders qualifiziert, die mit wässriger Formaldehydlösung (Formalin) zwei klar unterscheidbare Phasen ausbilden und kaum, bevorzugt kein Formalin lösen. Aus Tabelle 1 ist ersichtlich, dass hierfür besonders bevorzugt Kohlenwasserstoffe mit Kettenlängen von mindestens 8 C wie z.B. Isooctan, Dodecan oder Gemische daraus, aber auch Gemische wie Dieselkraftstoff geeignet sind. Bei Wahl dieser Extraktionsmittel ist das abgetrennte Kohlenwasserstoff-OMDAE-Gemisch ohne weitere Aufarbeitung, z.B. im Kraftstoffsektor, als Kraftstoff einsetzbar. In einer weiteren Ausgestaltung wird die organische Phase vor dem Einsatz weiter getrocknet, indem wasserdurchlässige bzw. -aufnehmende Mittel bevorzugt ausgewählt unter Polymermembranen, keramischen Membranen, Molekularsieben, Kieselgelen, Zeolithen bevorzugt ausgewählt unter Zeolith-A, MFI-Zeolith, und/oder wasserfreien Salzen bevorzugt ausgewählt unter Magnesiumsulfat, Calciumsulfat und/oder Calciumchlorid, eingesetzt werden, um Spuren von Wasser zu entfernen. In einer weiteren Ausgestaltung wird das Extraktionsmittel aus der organischen Phase entfernt, bevorzugt destillativ, und so das reine OMDAE-Gemisch, welches bei der Synthese entsteht, erhalten.

Die Komponenten der wässrigen Phase werden in einer Ausgestaltung nach Abtrennung des Wassers in das Reaktionsgefäß zurückgeführt und dort weiter umgesetzt. Die Entfernung, bzw. Die Komponenten der wässrigen Phase werden in einer Ausgestaltung nach Abtrennung des Wassers in das Reaktionsgefäß zurückgeführt und dort weiter umgesetzt. Die Entfernung, bzw. Reduktion der Menge an Wasser kann vorteilhaft über eine Membran erfolgen. In einer weiteren Ausgestaltung erfolgt die Reduktion bzw. Entfernung des Wassers aus der wässrigen Phase durch Standardprozesse, wie Gefriertrocknung, Verdunstung, Verdampfen, und/oder die Entfernung mittels Membranen, wie in WO 2013122268 A1, Tuan et al. J. Membr. Sci. 2002, 196, 111-123 und Rhim et al. J. Appl. Polym. Sci. 1998, 68, 1717-1723 beschrieben, und/oder die Entfernung des Wassers mittels wasserfreie Salze, Molekularsiebe, Kieselgele und/oder Zeolithe.

In einer Ausgestaltung, dem kontinuierlichen Betrieb, werden die mindestens einen Komponenten A und B einem Reaktionsgefäß zugeführt und dort mit dem mindestens einem Katalysator umgesetzt. Das resultierende Reaktionsgemisch wird mit dem Extraktionsmittel versetzt und im sich anschließenden Phasenseparator getrennt. Die organische Phase setzt sich aus den erwünschten OMDAE und dem Extraktionsmittel zusammen. Bei geeigneter Wahl des Extraktionsmittels, findet das abgetrennte Kohlenwasserstoff-OMDAE-Gemisch ohne weitere Aufarbeitung, z.B. im Kraftstoffsektor, Verwendung. Die Komponenten der wässrigen Phase werden nach Abtrennung des Wassers in das Reaktionsgefäß zurückgeführt und dort weiter umgesetzt. Die Entfernung des Wassers erfolgt vorteilhaft über eine Membran.

Eine weitere Ausgestaltung des Verfahrens ist die Durchführung der Reaktion in einem Zweiphasensystem bestehend aus wässriger und organischer Phase. Dabei gehen die erwünschten OMDAE unmittelbar nach der Reaktion in die organische Phase über, wodurch der Phasenseparator eingespart werden kann. Im Fall der OMDME-Synthese stellen sich die OMDME-Gehalte in der wässrigen bzw. organischen Phase dem Nernstschen Verteilungssatz entsprechend ein. Dabei wird unabhängig von der Menge an Extraktionsmittel das gleiche Produktspektrum erhalten.

In einer weiteren Ausgestaltung werden Kraftstoffe mit OMDAE direkt additiviert, indem der betreffende Kraftstoff direkt als Extraktionsmittel eingesetzt wird. Bei der Verwendung von Diesel wird dabei, sofern die Reaktion in einem Zweiphasensystem durchgeführt wird, auf einen separaten Extraktions- und Additivierungsschritt verzichtet, wodurch das Verfahren weiter vereinfacht und beschleunigt wird.

Die Löslichkeit der OMDAE in der' organischen Phase wird nicht nur durch Wahl des Extraktionsmittels sondern auch durch die chemische Modifikation der Endgruppen beeinflusst. So hat sich gezeigt, dass sich die Löslichkeit von OMDAE in Kohlenwasserstoffen beim Übergang von Methyl- zu Ethylethern oder höheren Alkylethern deutlich erhöht, so dass die Produkte in wesentlich größerem Umfang in die organische Phase überführt werden können. Durch die Variation der OMDAE-Endgruppen wird die Löslichkeit in organischen Lösungsmittel und Dieselkraftstoff eingestellt. Damit beeinflusst man zum einen den Übergang von OMDAE in die organische Phase und kontrolliert zum anderen die Eigenschaften der resultierenden Kohlenwasserstoff-OMDAE-Mischungen. Darüber hinaus werden durch die Wahl der Endgruppen auch die Eigenschaften der erzeugten Kohlenwasserstoff-OMDAE-Mischungen maßgeblich beeinflusst.

In einer weiteren Ausgestaltung werden gemischte Ether durch Verwendung von Alkoholgemischen erzeugt, wodurch das Phasenverhalten der Produkte ebenfalls beeinflusst werden kann. Ein erleichterter Übergang der OMDAE in die organische Phase begünstigt den Entzug der erwünschten Produkte aus dem Gleichgewichtsgemisch. Dies führt zu einer Steigerung des Umsatzes und damit zu einer weiteren Optimierung des Herstellungsverfahrens.

Die Gewinnung von OMDAE mit Hilfe eines Extraktionsverfahrens unter Vermeidung eines Destillationsschritts wurde bislang nicht beschrieben und die entsprechenden Vorteile waren nicht vorhersehbar. Da nicht umgesetzte Einsatzstoffe und unerwünschte Nebenprodukte effizient abgetrennt und wieder eingesetzt werden können, wird eine erhebliche Vereinfachung des Herstellungsverfahrens erreicht. Zur Gewinnung reiner Substanzen wird das beschriebene Verfahren in Kombination mit einer destillativen Trennung der gewonnenen Produktgemische in Einzelverbindungen durchgeführt.

Zielt die OMDAE-Gewinnung auf die Additivierung von Dieselkraftstoff, so kann der hier beschriebene Ansatz als besonders effizient angesehen werden. Da die OMDAE nicht als Reinstoffe mit einheitlicher Kettenlänge eingesetzt werden müssen, können OMDAE-Gemische vorteilhaft in einem wasserhaltigen Reaktionsgemisch erzeugt und direkt in die Dieselphase transferiert werden. Hierbei wird eine hohe Selektivität erreicht, d.h. Ausgangsstoffe und unerwünschte Nebenprodukte verbleiben in der wässrigen Reaktionsphase und können dort weiter umgesetzt werden.

Das Verfahren wird in einer weiteren Ausgestaltung zur Gewinnung von Reinstoffen eingesetzt. Dazu schließt sich an die Entnahme der organischen Phase eine Destillation an, um die Reinstoffe voneinander abzutrennen. Da Ausgangsstoffe und Nebenprodukte bereits im Extraktionsschritt abgetrennt wurden, erleichtert sich die Destillation. Im Fall der OMDME-Synthese werden leichtflüchtige Komponenten wie CH₃O-(CH₂O)ₙ-CH₃ mit n = 1 und 2 (Siedepunkte: 42 bzw. 105 °C) sehr einfach durch Erwärmen abgetrennt.

Die mittels des Verfahrens hergestellten OMDAE werden bevorzugt als Additive in Kraftstoffen, bevorzugt in Dieselkraftstoffen eingesetzt. Des Weiteren finden OMDAE als Lösungsmittel, Zündöle, Cetanbooster, Schmiermittel, als reiner Kraftstoff und Wachse Einsatz. Zündöle sorgen, durch Zusatz zu einem schwerzündenden Kraftstoffgemisch, für eine frühere Zündung. Sie erleichtern den Kaltstart und erhöhen die Motorleistung. Sie werden unter anderen in Blockheizkraftwerken eingesetzt. Cetanbooster werden Kraftstoffen hinzugefügt, um die Cetanzahl zu verbessern, das Kraftstoffsystem zu reinigen und es vor Korrosion zu schützen. Des Weiteren wirken sie gegen Bakterien und Pilze im Dieselkraftstoff und führen zu einer saubereren Verbrennung, was die Umwelt schützt. Bevorzugt finden Oxymethylethylether Einsatz als Zündöle, OMDME als Cetanbooster, OMDAE als Schmiermittel, OMDME mit n = 1 oder 2 als reiner Kraftstoff, bzw. als Ersatz für Benzin oder als Additiv in Benzin und OMDME mit n = 3 bis 5 als reiner Kraftstoff, bzw. als Ersatz für Diesel, OMDME mit n > 6 finden Einsatz als Wachse Einsatz. OMDME mit n > 6 sind fest und brennen rußfrei. Sie lassen sich daher für Kerzen, bevorzugt rußfreie Kerzen, einsetzen.

Die Erfindung wird im Folgenden anhand von Ausführungsbeispielen und den Figuren näher erläutert. Hierbei zeigen:
- **Fig. 1**: Fließschema zum Verfahren zur Herstellung von OMDAE-Gemischen
- **Fig. 2**: ein Fließschema zum Verfahren zur Herstellung von OMDAE-Gemischen im Zweiphasensystem
- **Fig. 3**: den Vergleich des Produktspektrums des Ausführungsbeispiels 1 V-1 (Reaktion mit anschließender Extraktion) mit dem von V-2 (Reaktivextraktion; FA = Formaldehyd, OME-OH = Halbacetale)
- **Fig. 4**: das Verhältnis des OMDME-Gehalts in der organischen Phase zum OMDME-Gehalt in der wässrigen Phase in Abhängigkeit von der Menge an Extraktionsmittel (siehe Ausführungsbeispiel 1 V-3)
- **Fig. 5**: die Produktspektren in der organischen und wässrigen Phase bei Verwendung von Diesel (DIN EN 590) als Extraktionsmittel (siehe Ausführungsbeispiel 1 V-4)
- **Fig. 6**: Vergleich des Produktspektrums von Ausführungsbeispiel 1 V-1 (a) mit dem von V-5 (b)

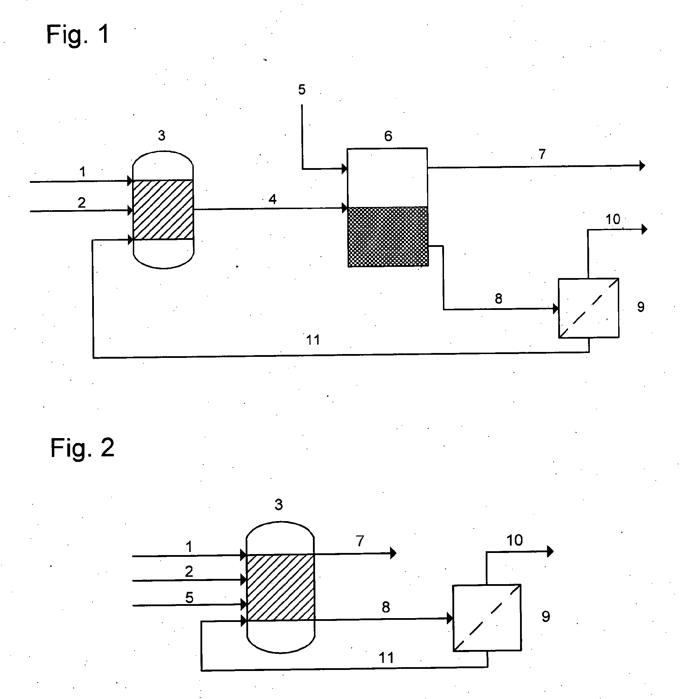

Eine Ausgestaltung des Verfahrens ist in **Figur 1** skizziert. Die mindestens eine Komponente A 1 und B 2 werden dem Reaktor 3 zugeführt und dort umgesetzt. Das resultierende Reaktionsgemisch 4 wird mit dem Extraktionsmittel 5 versetzt und im sich anschließenden Phasenseparator 6 getrennt. Die organische Phase 7 setzt sich aus den erwünschten OMDAE und dem Extraktionsmittel zusammen. Die Komponenten der wässrigen Phase 8 können nach Abtrennung des Wassers 10 in den Reaktor 3 zurückgeführt 11 und dort weiter umgesetzt werden. Die Entfernung des Wassers kann vorteilhaft über eine Membran 9 erfolgen.

Eine weitere Ausgestaltung des Herstellungsverfahrens ist in **Figur 2** gezeigt. Die mindestens eine Komponente A 1 und B 2 werden dem Reaktor 3 zugeführt und dort umgesetzt. Das resultierende Reaktionsgemisch wird mit dem Extraktionsmittel 5 versetzt. Indem die Reaktion in einem Zweiphasensystem bestehend aus wässriger 8 und organischer Phase 7 durchgeführt wird, gehen die erwünschten OMDAE unmittelbar nach der Reaktion in die organische Phase 7 über, wodurch der Phasenseparator (Einheiten 4 und 6 in Figur 1) eingespart wird. Die organische Phase 7 setzt sich aus den erwünschten OMDAE und dem Extraktionsmittel zusammen. Die Komponenten der wässrigen Phase 8 können nach Abtrennung des Wassers 10 in den Reaktor 3 zurückgeführt 11 und dort weiter umgesetzt werden. Die Entfernung des Wassers kann vorteilhaft über eine Membran 9 erfolgen.

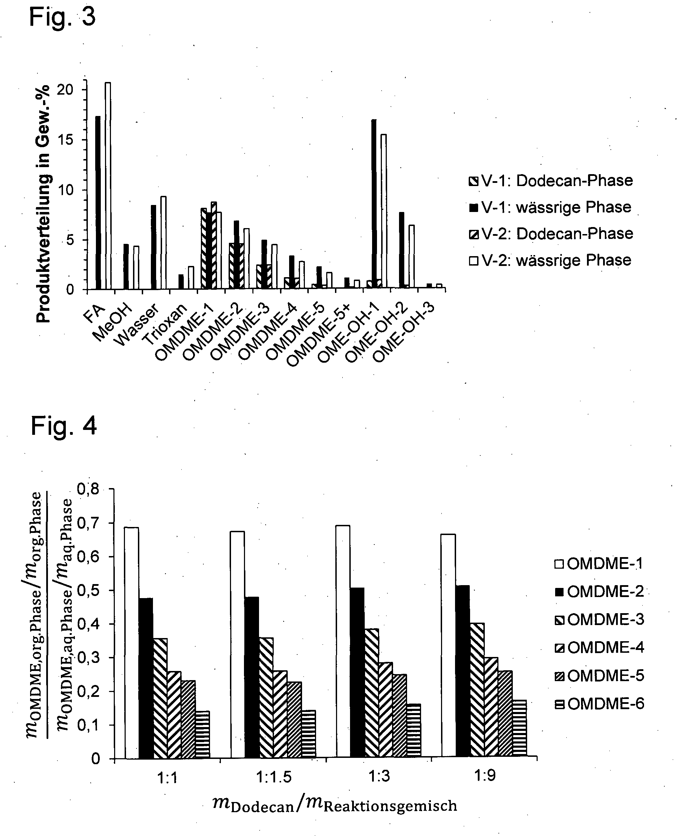

Eine Gegenüberstellung der Ergebnisse der Ausführungsbeispiele 1 V-1 und V-2 findet sich in **Figur 3****.** Die Verteilung der OMDAE in der organischen und wässrigen Phase ist nahezu unabhängig von der Versuchsdurchführung. Die Reaktion mit anschließender Extraktion (V-1) und Extraktivreaktion (V-2) liefern ähnliche Ergebnisse. Damit bietet das in Figur 2 beschriebene Verfahren weitere erhebliche Vorteile. Die OMDME-Gehalte in der wässrigen bzw. organischen Phase stellen sich dem Nernstschen Verteilungssatz entsprechend ein. Dabei wird, wie aus **Figur 4** ersichtlich, unabhängig von der Menge an Extraktionsmittel das gleiche Produktspektrum erhalten.

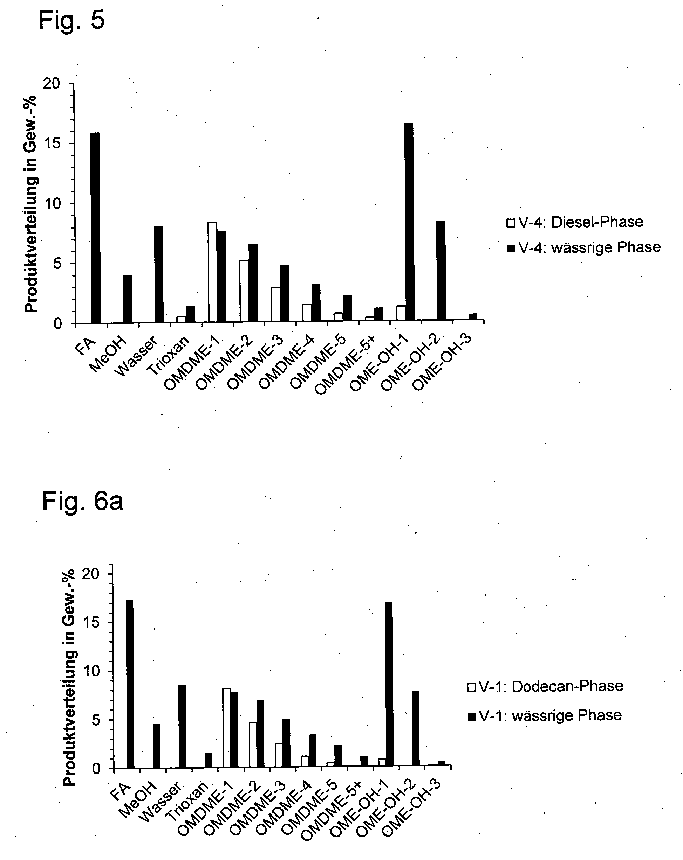

Die Ergebnisse des Ausführungsbeispiels V-4 sind in **Figur 5** zusammengefasst. Dabei wurde die Reaktion wie unter V-1 beschrieben durchgeführt und es wurde anstatt Dodecan Dieselkraftstoff gemäß DIN EN 590 als Extraktionsmittel eingesetzt. Wie aus Figur 5 hervorgeht, verbleiben sowohl die Edukte als auch unerwünschte Nebenprodukte in der wässrigen Phase wohingegen die erwünschten OMDME sehr selektiv in die Dieselphase übergehen. Damit kann die Reaktion vorteilhaft in einem Zweiphasensystem aus wässriger Reaktionsphase und Dieselphase als Extraktionsmedium durchgeführt werden. Ein Additivierungsschritt entfällt und die Dieselphase kann direkt als Kraftstoff eingesetzt werden.

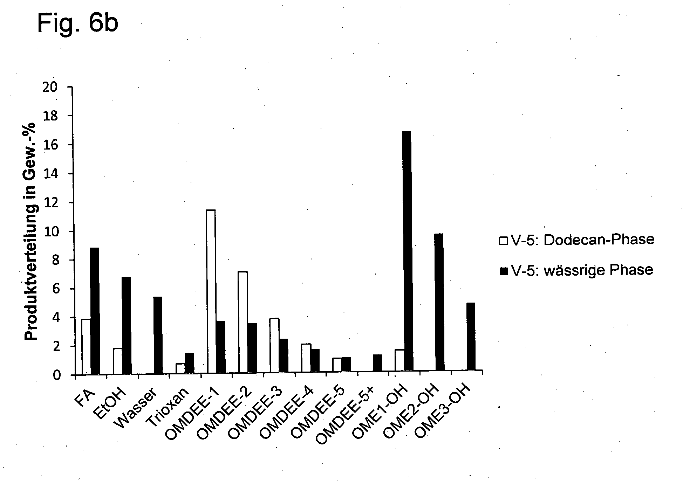

Wie aus **Figur 6a** ersichtlich ist, verbleiben erhebliche Mengen der erwünschten Produkte (OMDME) in der wässrigen Phase. Dem kann durch Synthese der Oxymethylendiethylether (OMDEE, CH₃CH₂O-(CH₂O)ₙ-CH₂CH₃, n ≈ 1 - 6) entgegengewirkt werden (**Figur 6b**), wodurch eine weitere deutliche Effizienzsteigerung im Herstellungsprozess erreicht wird. Prinzipiell können auch gemischte Ether durch Verwendung von Alkoholgemischen erzeugt werden, wodurch das Phasenverhalten der Produkte ebenfalls beeinflusst werden kann. Ein erleichterter Übergang der OMDAE in die organische Phase begünstigt den Entzug der erwünschten Produkte aus dem Gleichgewichtsgemisch. Dies kann zu einer Steigerung des Umsatzes und damit zu einer weiteren Optimierung des Herstellungsverfahrens führen.

### Ausführungsbeispiel 1: Herstellung oligomerer OMDME

V-1: Es werden 60,0 g (2,00 mol) *p*-Formaldehyd, 40,0 g (1,25 mol) Methanol und 1,0 g des Katalysators Amberlyst 36 bei 80 °C in einem Autoklaven für 24 h zur Reaktion gebracht. 10 g des Produktgemischs werden filtriert und mit 10 g Dodecan extrahiert. Die beiden Phasen werden anschließend gaschromatographisch analysiert. Der Anteil an OMDME im Extraktionsmittel beträgt 13,4 Gew.-%.
V-2: 60,0 g (2,00 mol) *p*-Formaldehyd, 40,0 g (1,25 mol) Methanol, 100,0 g Dodecan und 1,0 g Amberlyst 36 werden bei 80 °C in einem Autoklaven für 24 h umgesetzt. Nach Filtration des Produktgemischs werden beide Phasen separiert und gaschromatographisch analysiert. Der Anteil an OMDME im Extraktionsmittel beträgt 12,7 Gew.-%.
V-3: 60,0 g (2,00 mol) *p*-Formaldehyd, 40,0 g (1,25 mol) Methanol und 1,0 g des Katalysators Amberlyst 36 werden bei 80 °C in einem Autoklaven für 24 h zur Reaktion gebracht. Danach wird das Reaktionsgemisch mit Dodecan extrahiert. Es werden vier Versuche durchgeführt, wobei die Massenverhältnisse von Dodecan zu Reaktionsgemisch 1:1, 1:1,5, 1:3 und 1:9 betragen. Der Anteil an OMDME im Extraktionsmittel beträgt dann jeweils 13,2, 14,0, 15,9 und 16,2 Gew.-%.
V-4: 60,0 g (2,00 mol) *p*-Formaldehyd, 40,0 g (1,25 mol) Methanol und 1,0 g Amberlyst 36 werden bei 80 °C für 24 h in einem Autoklaven umgesetzt. 10 g des so erhaltenen Reaktionsgemischs werden mit 10 g Diesel (DIN EN 590) extrahiert und analysiert. Der Anteil an OMDME im Extraktionsmittel beträgt 14,1 Gew.-%.
V-5: 60,0 g (2,00 mol) *p*-Formaldehyd, 57,4 g (1,25 mol) Ethanol und 0,6 g Amberlyst 36 werden bei 100 °C für 24 h in einem Autoklaven umgesetzt. 10,0 g der erhaltenen Probe werden mit 10,0 g Dodecan extrahiert und die so erhaltenen Phasen werden gaschromatographisch analysiert. Der Anteil an OMDEE im Extraktionsmittel beträgt 17,8 Gew.-%.

### Ausführungsbeispiel 2: Ermittlung geeigneter Extraktionsmittel

Zur Bestimmung der Löslichkeit von Edukten und Produkten in' unterschiedlichen Lösungsmitteln wurde der zu lösende Stoff schrittweise zu einer definierten Menge Lösungsmittel gegeben bis der zu lösende Stoff und das Lösungsmittel im volumetrischen Verhältnis von 1:1 vorlagen. Die Versuche wurden bei Raumtemperatur und unter Atmosphärendruck durchgeführt. Es wurde mehrmals geschüttelt und die Löslichkeit/Phasentrennung nach einigen Minuten visuell bestimmt. Während des Mischungsvorgangs wurde das Lösungsverhalten beobachtet und wie folgt klassifiziert:
- P: Phasenbildung
- l: löslich
- ++: Stoff ist sehr gut mischbar; Löslichkeit in beliebigen Mengen.
- +: Stoff ist gut löslich; Löslichkeit nicht beliebig.
- 0: Stoff löst sich erst nach mehrmaligem Schütteln; Begrenzte Löslichkeit.
- -: Stoff ist schlecht löslich; Es löst sich nur ein geringer Anteil im Lösungsmittel.
- --: Stoff ist unlöslich; Keine Löslichkeit erkennbar.

**Tabelle: Löslichkeiten von OMDAE, Alkoholen und Formalin in verschiedenen Lösungsmitteln.**

| Extraktionsmittel | X | Y/C | D | E | F | G | H | I | J | |
|---|---|---|---|---|---|---|---|---|---|---|
| | | | | | | | | | l | P |
| Petrolether | + | + | 0 | - | + | + | 0 | + | ~50% | ja |
| Pentan | + | + | -- | - | + | + | 0 | + | ~50% | ja |
| n-Hexan | + | + | -- | - | + | + | 0 | + | ~50% | ja |
| Cyclohexan | + | + | - | - | + | + | 0 | + | ~30% | ja |
| Heptan | + | + | -- | - | + | + | 0 | + | ~30% | ja |
| Isooctan | + | + | -- | -- | + | + | 0 | + | - | ja |
| Dodecan | + | + | -- | -- | + | + | 0 | + | -- | ja |
| Diesel | + | + | -- | - | + | + | 0 | + | -- | ja |
| Toluol | + | + | + | + | + | + | + | + | -- | ja |
| DPE | + | + | 0 | ++ | + | + | 0 | + | - | ja |
| DBE | + | + | 0 | + | + | + | 0 | + | - | ja |
| DEE | + | + | - | + | + | + | + | ++ | + | nein |
| 1,4-Dioxan | + | + | + | + | + | + | + | + | + | nein |
| THF | + | + | 0 | + | + | + | + | + | + | nein |
| MeOH | + | + | - | + | + | + | + | ++ | + | nein |
| Aceton | ++ | ++ | ++ | ++ | ++ | ++ | ++ | ++ | ++ | nein |
| Chlorbenzol | + | + | + | + | + | + | + | + | ++ | nein |
| Dichlorethan | 0 | + | + | ++ | + | + | + | + | - | ja |
| Chloroform | + | + | ++ | ++ | + | + | + | + | ~30% | ja |
| Dichlormethan | ++ | ++ | ++ | ++ | + | + | + | + | ~30% | ja |
| Nitrobenzol | ++ | ++ | ++ | + | + | + | + | + | - | ja |
| DMSO | + | + | 0 | + | + | + | + | + | + | nein |
| Ölsäuremethylester | ++ | ++ | - | + | + | + | - | + | - | ja |
| Benzol | + | + | + | + | + | + | + | + | -- | ja |
| Glyme | + | + | 0 | + | + | + | + | + | + | nein |
| DMF | + | + | + | + | + | + | + | + | + | nein |
| Benzylalkohol | + | + | + | + | + | + | 0 | + | + | nein |

| | | | | | | | | | | |
|---|---|---|---|---|---|---|---|---|---|---|
| X: Oxymethylendimethylether CH₃O-(CH₂O)ₙ-CH₃ mit n = 1; Y: Oxymethylendimethylether CH₃O-(CH₂O)ₙ-CH₃ mit n = 3; C: Oxymethylendimethylether CH₃O-(CH₂O)ₙ-CH₃ mit n = 4; Y/C: 50 Gew.-% Y + 50 Gew.-%. C; D: Oxymethylendimethylether CH₃O-(CH₂O)ₙ-CH₃ mit n = 6; E: Methanol; F: Oxymethylendiethylether CH₃CH₂O-(CH₂O)ₙ-CH₂CH₃ mit n = 1; G: Oxymethylendiethylether CH₃CH₂O-(CH₂O)ₙ-CH₂CH₃ mit n = 3; H: Oxymethylendiethylether CH₃CH₂O-(CH₂O)ₙ-CH₂CH₃ mit n = 6; I: Ethanol; J: 38 Gew.-% Formaldehyd in Wasser; Diesel = Dieselkraftstoff gemäß DIN EN 590; DPE = Di-*i*-propylether; DBE = Di-*n*-butylether; DEE = Diethylether. | | | | | | | | | | |

Sollten sich zwei homogene Phasen bilden, wird Phasenbildung (P) mit "ja" bezeichnet. Sollten sich keine klaren Phasengrenzen ergeben, dann mit "nein".

### Referenzen

Tuan et al. J. Membr. Sci. 2002, 196, 111-123
Rhim et al. J. Appl. Polym. Sci. 1998, 68, 1717-1723

## Patentansprüche

1. Verfahren zur Herstellung oligomerer Oxymethylendialkylether (OMDAE)
mit der Formel
R¹O-(CR³R⁴-O)ₙ-R² (I)
mit R¹, R² = Alkyl, Cycloalkyl, Acyl und
R³, R⁴ = Alkyl, Cycloalkyl, Wasserstoff, wobei die Reste R¹, R²,
R³ und/oder R⁴ Heteroatome aufweisen können und
R¹ =/≠ R² =/≠ R³ =/≠ R⁴
und n = ganze Zahl ≥ 1,
mit den folgenden Verfahrensschritten:
a) Bereitstellen mindestens einer Komponente A und mindestens einer Komponente B, wobei A ein Alkohol und/oder eine Carbonsäure und B ein Aldehyd und/oder ein Keton ist,
b) Umsetzung der mindestens einen Komponenten A und B mit mindestens einem sauren Katalysator,
c) Erzeugung einer wässrigen und einer organischen Phase,
d) Entnahme der organischen Phase,
wobei in Verfahrensschritt c) als Extraktionsmittel mindestens Diesel hinzugegeben wird.

2. Verfahren gemäß Anspruch 1, wobei nach Verfahrensschritt d) folgende Verfahrensschritte erfolgen:
d i) Trocknen der organischen Phase,
d ii) Entfernen des mindestens einem Extraktionsmittel aus der organischen Phase,
d iii) Entnahme des mindestens einem OMDAE nach Formel (I).

3. Verfahren gemäß Anspruch 1 oder 2 zur Herstellung oligomerer OMDAE mit R¹, R² = Methyl, Ethyl, Propyl, und R¹ =/≠ R².

4. Verfahren gemäß einem der Ansprüche 1 bis 3 zur Herstellung oligomerer OMDAE mit n = 1, 2, 3, 4, 5, 6.

5. Verfahren gemäß einem der Ansprüche 2 bis 4, wobei das Trocknen der organischen Phase im Verfahrensschritt d i) mit mindestens einem Trockenmittel, ausgewählt aus der Liste aus Polymermembranen, keramische Membranen, Molekularsieben, Kieselgelen, Zeolithen und/oder wasserfreien Salzen ausgewählt unter Magnesiumsulfat, Calciumsulfat und/oder Calciumchlorid durchgeführt wird.

6. Verfahren gemäß einem der Ansprüche 1 bis 5, wobei nach Verfahrensschritt c) folgende Verfahrensschritte erfolgen:
c i) Entnahme der wässrigen Phase,
c ii) Reduktion des Wassergehaltes der wässrigen Phase,
c ii) Rückführung der wässrigen Phase in das Reaktionsgefäß.

7. Verfahren gemäß Anspruch 6 wobei die Reduktion des Wassergehalts der wässrigen Phase im Verfahrensschritt c ii) mit mindestens einem Verfahren, ausgewählt aus der Liste aus Gefriertrocknung, Verdunstung, Verdampfen, und/oder die Entfernung mittels Membranen, wasserfreien Salzen, Molekularsieben, Kieselgelen und/oder Zeolithen, erfolgt.

## Claims

1. Method for the preparation of oligomeric oxymethylene dialkyl ethers (OMDAE) with the formula
R¹O-(CR³R⁴-O)ₙ-R² (I)
where R¹, R² = alkyl, cycloalkyl, acyl and
R³, R⁴ = alkyl, cycloalkyl, hydrogen, whereby the residues R¹, R²,
R³ and/or R⁴ can comprise heteroatoms, and
R¹ =/≠ R² =/≠ R³ =/≠ R⁴
and n = whole number ≥ 1,
with the following method steps:
a) providing at least one component A and at least one component B, with A being an alcohol and/or a carboxylic acid and B an aldehyde and/or a ketone,
b) conversion of the at least one components A and B with at least one acid catalyst,
c) generating an aqueous and an organic phase,
d) removing the organic phase,
with at least diesel being added as extraction agent in method step c).

2. Method of claim 1 in which the following method steps are carried out after method step d):
d i) drying of the organic phase,
d ii) removing of the at least one extraction agent from the organic phase,
d iii) withdrawing the at least one OMDAE according to formula (I).

3. Method of claim 1 or 2 for the preparation of oligomeric OMDAE with R¹, R² = methyl, ethyl, propyl, and R¹ =/≠ R².

4. Method of any one of claims 1 to 3 for the preparation of oligomeric OMDAE with n = 1, 2, 3, 4, 5, 6.

5. Method of any one of claims 2 to 4, with the drying of the organic phase in method step d i) being carried out with at least one drying agent selected from the list of polymer membranes, ceramic membranes, molecular sieves, silica gels, zeoliths and/or anhydrous salts selected from magnesium sulphate, calcium sulphate and/or calcium chloride.

6. Method of any one of claims 1 to 5 in which the following method steps are carried out after method step c):
c i) withdrawal of the aqueous phase,
c ii) reduction of the water content of the aqueous phase,
c ii) returning the aqueous phase into the reaction vessel.

7. Method of claim 6, with the reduction of the water content of the aqueous phase in method step c ii) being effected with at least one method selected from the list of freeze drying, evaporation, vaporisation, and/or the removal by way of membranes, anhydrous salts, molecular sieves, silica gels and/or zeoliths.

## Revendications

1. Procédé de préparation d'oxyméthylène dialkyléther (OMDAE) oligomères
de formule
R¹O-(CR³R⁴O)ₙ-R² (I)
dans laquelle R¹ et R² représentent un alkyl, un cycloalkyl, ou un acyl et
R³ et R⁴ représentent un alkyl, un cycloalkyl ou un hydrogène,
les gourpes R¹, R², R³ et/ou R⁴ pouvant renfermer des hétéroatomes, et
R¹ =/≠ de R² =/≠ R³ =/≠ de R⁴, et
n représente un nombre entier ≥1,
procédé comprenant les étapes suivantes consistant à :
a) préparer au moins un composé A et au moins un composé B, A étant un alcool et/ou un acide carboxylique et B étant un aldéhyde et/ou une cétone,
b) faire réagir le composant A et le composant B avec au moins un catalyseur acide,
c) obtenir une phase aqueuse et une phase organique,
d) prélever la phase organique,
lors de l'étape c), en tant qu'agent d'extraction au moins un gazole étant ajouté.

2. Procédé conforme à la revendication 1,
selon lequel après l'étape d) on met en oeuvre les étapes suivantes consistant à :
d i) sécher la phase organique,
d ii) éliminer l'agent d'extraction de la phase organique,
d iii) prélever l'OMDAE selon la formule (I).

3. Procédé conforme à la revendication 1 ou 2,
de préparation d'OMDAE oligomères selon lequel R¹et R² représentent un méthyl, un éthyl ou un propyl, et R¹ =/≠ de R².

4. Procédé de préparation l'OMDAE oligomères conforme à l'une des revendications 1 à 3, selon lequel n = 1,2,3,4,5,6.

5. Procédé conforme à l'une des revendications 2 à 4,
selon lequel le séchage de la phase organique lors de étape d i) est effectuée à l'aide d'au moins un agent de séchage choisi dans la liste formée par des membranes polymères, des membranes céramiques, des tamis moléculaires , des gels de silice , des zéolithes et/ou des sels anhydres, choisis parmi le sulfate de magnésium, le sulfate de calcium et/ou le chlorure de calcium.

6. Procédé conforme à l'une des revendications 1 à 5,
selon lequel après l'étape c) on met en oeuvre les étapes suivantes consistant à :
c i) prélever le phase aqueuse,
c ii) réduire la teneur en eau de la phase aqueuse,
c ii) recycler la phase aqueuse dans le réacteur.

7. Procédé conforme à la revendication 6,
selon lequel la réduction en teneur eau de la phase aqueuse lors de l'étape c ii) est effectuée à l'aide d'au moins un procédé choisi dans une liste formée par une lyophilisation , une évaporation, une vaporisation, et/ou une élimination au moyen de membranes de sels d'eau, exempts de tamis moléculaires, de gels de silice et/ou de zéolithes.
